# EUROPEAN PATENT SPECIFICATION

(11) **EP 3 250 180 B1**
(45) Date of publication and mention of the grant of the patent: **11.03.2026**
(21) Application number: 16701782.1
(22) Date of filing: 27.01.2016
(51) Int. Cl.: A61Q 19/00, A61K 8/67, A61K 31/122, A23L 33/15, A23L 33/18, A61K 38/39

(54) **PHARMACEUTICAL COMPOSITION COMPRISING VITAMIN K2 FOR USE IN THE TREATMENT OR PREVENTION OF MELANOMA**
PHARMAZEUTISCHE ZUSAMMENSETZUNG MIT VITAMIN K2 ZUR VERWENDUNG BEI DER BEHANDLUNG ODER VORBEUGUNG VON MELANOMEN
COMPOSITION PHARMACEUTIQUE COMPRENANT DE LA VITAMINE K2 POUR LE TRAITEMENT OU LA PREVENTION DU MELANOME

(30) Priority: 27.01.2015 EP 15152764
(43) Date of publication of application: 06.12.2017
(73) Proprietor: Lesaffre et Compagnie, 75001 Paris (FR)
(72) Inventor: VERMEER, Cornelis, 6229 EV Maastricht (NL)
(74) Representative: Acapo Onsagers AS
(86) International application number: PCT/EP2016/051724
(87) International publication number: WO 2016/120341

(56) References cited:
- EP-A1- 2 561 869
- WO-A1-2012/000930
- WO-A1-2014/017145
- WO-A2-02/02764
- WO-A2-2006/113479
- WO-A2-2009/063485
- US-A1- 2007 142 462
- US-A1- 2010 247 693
- PRASAD K N ET AL: "Vitamin K"3 (menadione) inhibits the growth of mammalian tumor cells in culture", LIFE SCIENCE, PERGAMON PRESS, OXFORD, GB, vol. 29, no. 13, 28 September 1981 (1981-09-28), pages 1387 - 1392, XP023722824, ISSN: 0024-3205, [retrieved on 19810928], DOI: 10.1016/0024-3205(81)90683-4
- SCHURGERS LEON J ET AL: "Matrix Gla-protein: The calcification inhibitor in need of vitamin K", THROMBOSIS AND HAEMOSTASIS, SCHATTAUER GMBH, DE, vol. 100, 1 October 2008 (2008-10-01), pages 593 - 603, XP002657395, ISSN: 0340-6245, [retrieved on 20080905], DOI: 10.1160/TH08-02-0087

## Description

### Field of the invention

This invention relates to the fields of cosmetics and dermatology and discloses the beneficial action of vitamin K and vitamin K-related peptides on skin health, notably on a) epidermal skin health and the barrier function of the stratum granulosum, and b) counteracting the growth and progression of skin cancer, including melanoma. Therapeutic and non-therapeutic methods for treatment or prevention as well as pharmaceutical, nutritional, cosmetic and dermatological compositions are disclosed which counteract malign cell growth, improve skin repair, counteract skin ageing and support the barrier function of the healthy skin.

### Background art

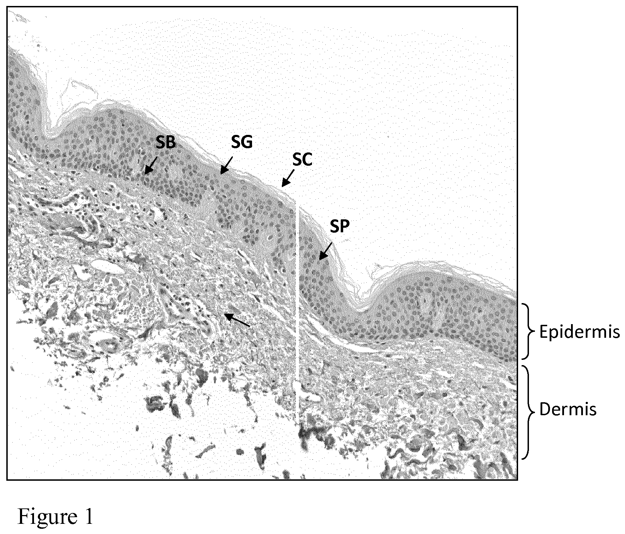

The healthy skin is built of several layers, and Figure 1 shows the general skin architecture. The lower layer is called the dermis, where fibroblasts deposit connective tissue fibers (collagen and elastin) which are important for skin strength. The upper layer is called epidermis and its most important function is to serve as a barrier between the body and the outer environment. Cells found in the epidermis are: keratin ocytes, melanocytes, Langerhans cells and Merkel cells. The epidermis can be subdivided further into different layers: stratum basale, stratum granulosum, stratum corneum, and stratum spinosum. The stratum granulosum contains a calcium gradient, which has an important role in the barrier function of the epidermis [Elias, P.M. et al. J. Invest. Dermatol. 2002; 119:1128-2236]
The only well-described function of vitamin K is to serve as a cofactor for the enzyme γ-glutamate carboxylase (GGCX) during the posttranslational carboxylation of glutamate residues into γ-carboxyglutamate (Gla) [Berkner, K.L. Vitam. Horm. 2008; 78: 131-156]. Proteins undergoing this carboxylation are known as Gla-proteins. Currently 17 Gla-proteins have been identified, one of which is Matrix Gla-Protein (MGP) [Theuwissen, E. et al. Adv. Nutr. 2012; 3: 166-173]. In all cases in which their function is known, the carboxylation step is a prerequisite for their biological activity. It has been demonstrated that GGCX occurs abundantly in skin, both in the dermis and in the epidermis [de Boer-van den Berg, M.A.G. et al. J. Invest. Dermatol. 1986; 87: 377-380]. MGP, a strong calcification inhibitor, has been found associated with the elastic fibers in the dermis [Gheduzzi, D. et al. Lab Invest. 2007; 87: 998-1008], where it is synthesized by the fibroblasts [Boraldi, F. et al. J. Invest. Dermatol. 2013; 133: 946-954]. A function for GGCX in the epidermis has not yet been found thus far.

Different functions have been reported for the various Gla-proteins: the most well-known are those involved in hemostasis, such as prothrombin and the clotting factors VII, IX and X [Cranenburg, E.C.M. et al. Thromb. Haemostas. 2007; 98: 120-125]. Another family of Gla-proteins is that of the calcification inhibitors: osteocalcin [Price, P.A. Vitam. Horm. 1985; 42: 65-108], Matrix Gla-Protein [Schurgers L.J. et al. Thromb. Haemostas. 2008; 100: 593-603] and Gla-rich Protein (GRP, also known as UCMA) [Viegas, C.S. et al. Am. J. Pathol. 2009; 175: 2288-2298]. Mature osteocalcin contains three Gla-residues, is synthesized by the osteoblasts in bone and has a role in the proper alignment of the calcium phosphate crystals to provide optimal bone strength. Recently, a second function for osteocalcin has been reported, notably that of a hormone stimulating both fat metabolism in adipocytes and male fertility [Karsenty, G. C. R. Biol. 2011; 334: 720-224]. The most remarkable fact is that in order to become an active hormone, osteocalcin has first to be decarboxylated [Clemens, T.L. and Karsenty, G. J. Bone Miner. Res. 2011; 26: 677-680]. Thus, high vitamin K intake resulting in maximal osteocalcin carboxylation is also associated with maximal production of decarboxylated osteocalcin and increased fat metabolism [WO 2012/080519 A1 ].

MGP is another well-known Gla-protein. It is the strongest inhibitor of ectopic soft tissue calcification and occurs abundantly in cartilage [Hale, J.E. et al. J. Biol. Chem. 1988; 263: 5820-5824] and arterial vessel wall [Wallin, R. et al. Thromb Haemostas. 1999; 82: 1764-1767]. MGP was also found in skin where it is protects collagen and elastin fibers against calcification [Gheduzzi, D. et al. Lab Invest. 2007; 87: 998-1008]. Besides its 5 Gla-residues, mature MGP also contains 3 serine residues that may be phosphorylated, but the function of these phosphoserine residues is presently unknown.

As was demonstrated by Price, these small calcification inhibitors are essential in the prevention of fibril calcification, a principle that was designated as mineralization by inhibitor exclusion [Price, P.A. et al. J Biol Chem. 2009; 284: 17092-17101]. Collagen and elastin, however, only occur in the dermis, not in the epidermis, as was demonstrated by multiphoton microscopic imaging [Zhu, Z et al. Scanning 2014 (epub)].

The epidermis is the outermost layer of skin serving as an impervious biological layer with functions of barrier. The changes of epidermal thickness and the morphological alteration in stratum granulosum, stratum corneum or stratum spinosum are tightly associated with skin diseases and barrier dysfunction such as the psoriasis, parakeratosis, atopic dermatitis or contact dermatitis, scars and fissures. Within the stratum granulosum a calcium gradient strongly contributes to this barrier function, and it has been demonstrated that disturbances in the calcium gradient result in impaired expression of differentiation-specific proteins (such as loricrin); modulations in epidermal calcium coordinately regulate epidermal differentiation that together form said barrier [Elias, P.M. et al. J. Invest. Dermatol. 2002;119:1128-1136]. A healthy epidermis is therefore of utmost importance for skin health and for protection against intrusion by foreign substances and microorganisms.

Another well-known Gla-protein is Gas6 (growth-arrest-specific gene 6 protein), one of the functions of which is to serve as a ligand for members of the TAM receptor tyrosine kinase family, and in this way to participate in cell growth regulation [see for instance: Axelrod, H. and Pienta, K.J. Axl as a mediator of cellular growth and survival. Oncotarget. 2014, 15;8818-8852]. A role distinct from γ-glutamate carboxylation has also been described for vitamin K, notably MK-4, as a ligand for the nuclear membrane receptor: steroid and xenobiotic receptor SXR, which forms a second pathway in which vitamin K is involved in the regulation of cell growth [see for instance: Azuma, K., Ouchi, Y., Inoue, S. Vitamin K: novel molecular mechanisms of action and its roles in osteoporosis. Geriatr. Gerontol. Int. 2014, 14, 1-7]. In this way it can be understood that tumor suppressive effects have been found for menaquinone-4 in several tissues, for instance in hepatocellular carcinoma [see for instance: Li, L, Qi, Z., Qian, J., Bi, F., Lv, J., Xu, L, Zhang, L, Chen, H., Jia, R. Induction of apoptosis in hepatocellular carcinoma Smmc-7721 cells by vitamin K(2) is associated with p53 and independent of the intrinsic apoptotic pathway. Mol. Cell. Biochem. 2010, 342, 125-131]. Antiproliferative effects on skin cancer, notably melanoma, have not been reported for K vitamins, but in cell culture it was demonstrated that the synthetic analog menadione (a molecule without vitamin K activity), may inhibit melanoma proliferation in cell culture. For natural forms of vitamin K (phylloquinone and menaquinones) such activity was not found or not reported [Ishibashi, M., Arai, M., Tanaka, S., Onda, K., Hirano, T. Antiproliferative and apoptosis-inducing effects of lipophilic vitamins on human melanoma A375 cells in vitro. Biol. Pharm. Bull. 2012;35, 10-17].

In the scientific literature, the use of vitamin K for skin health has been described for treating bruises [see for instance Shah, N.S. et al. J. Am. Acad. Derm. 2002;47:241-244], but the mechanism behind this putative effect remains unclear, and the efficacy is highly disputed [see for instance Kovacs R.K. et al. J. Am. Acad. Dermatol. 2004;50:982-983]. Moreover, topical application of vitamin K (in some cases in combination with vitamin D) has been reported to improve laser-induced purpura of the skin [Leu, S. et al. Brit. J. Dermatol. 2010; 16:557-563 and: Lou, W.W. et al. Dermatol. Surg. 1999;25:942-944] and wound healing [Hemmati, A.A. et al. Indian J. Pharmacol 2014;46:409-412]. Nanoencapsulation of vitamin K was reported to improve uptake in the skin [Campari, V. et al. Int. J Nanomed. 2014;9:1823-1832 and da Silva, A.L. et al. Skin Res. Technol. 2013;19:e223-e230]. Specific effects of vitamin K on epidermal health or functions have not been reported so far.

In the patent literature, the use of vitamin K for treating various skin conditions has been described.

WO 2006/1 13479 A2 discloses vitamin K1 or vitamin K3 for use in a method for treating or preventing a skin rash secondary to anti-epidermal growth factor receptor (EGFR) therapy in cancer patients. EGFR inhibitors were shown to have antitumor activity in a variety of solid tumors. Clinical benefits defined as relief of symptoms or prolonged survival have been demonstrated with anti-EGFR antibodies, but the major side effect of this therapy is skin rash, itching and secondary infections. These symptoms were reported to be relieved by topical application of vitamin K1 or vitamin K3. Remarkably, the most prominent effect was found for menadione (i.e. vitamin K3, a synthetic analog not having the classical vitamin K activity), with some effect for vitamin K1 and no effect at all for vitamin K2.

WO 2009/063485 A2 discloses the use of vitamin K for (1 ) improved blood perfusion and in this way ameliorating hypoxia in venous insufficiency; (2) hyper- pigmentation of the skin; and (3) vascular problems of the skin.

US 2010/0247693 A1 discloses the use of vitamin K for treating rosacea. Rosacea is a chronic inflammatory disorder of the skin associated with erythema (redness) caused by dilatation of superficial blood vessels in the skin. Vitamin K is mentioned as a compound alleviating rosacea.

EP 2561869 A1 mentions the use of vitamin K as a promotor of skin collagen. Collagen is synthesized in the dermis (not in the epidermis) by fibroblasts. Remarkably, this activity was only observed for menaquinone-7 (MK-7) and not for other forms of vitamin K.

Further prior art includes WO 2006/113479 disclosing a method for treating a skin rash comprising applying a vitamin K analog topically, WO 2014/017145 disclosing an oral or topical composition comprising vitamin K analogs as active compounds for improving skin function, and US2007/142462 disclosing a pharmaceutical composition for use in the treatment or prevention of skin cancer in a mammalian subject.

From PRASAD K N et al: "Vitamin K"3 (menadione) inhibits the growth of mammalian tumor cells in culture", LIFE SCIENCE, PERGAMON PRESS, OXFORD, GB, vol. 29, no. 13, 28 September 1981, pages 1387-1392, XP023722824, ISSN: 0024-3205, DOI: 10.1016/0024-3205(81 )90683-4 it is known that vitamin K inhibits the growth of mouse melanoma.

It is an object of the present invention to provide certain new uses of vitamin K, in particular the commercially available forms K1 , MK-4 and MK-7, and peptides containing the vitamin K-dependent amino acid γ-carboxyglutamate (Gla), in particular MGP and MGP-derived peptides, which are applicable to the general population (not restricted to patients) based on new effects which were observed in cultured skin systems in the absence of blood vessels and in the epidermis.

### Summary of the invention

The present invention relates to a pharmaceutical composition for use in treatment or prevention of melanoma in a human subject, wherein the composition comprises a therapeutically effective amount of vitamin K2.

In the following it will be described providing of vitamin K1 , vitamin K2, Matrix Gla-Protein (MGP) and bioactive peptides derived from MGP, for use in a method for treating or preventing skin cancer, in particular melanoma, parakeratosis, improving the skin integrity, the skin barrier function and/or decreasing skin ageing in a mammalian subject, preferably a human subject, the method comprising applying said vitamin K1, vitamin K2, MGP or MGP-derived peptides to the skin in an amount effective to treat skin cancer or parakeratosis, to improve the skin integrity, the skin barrier function and/or to decrease skin ageing, wherein said vitamin K1 , vitamin K2, MGP or MGP-derived peptides are formulated in a formulation suitable for topical administration.

It will further be described use of vitamin K1, vitamin K2, MGP and bioactive peptides derived from MGP in the manufacture of a pharmaceutical, nutraceutical, cosmetic or dermatological composition or treating or preventing skin cancer, in particular melanoma, parakeratosis, improving the skin integrity, the skin barrier function and/or decreasing skin ageing in a mammalian subject, preferably a human subject, wherein said composition is formulated for topical administration and comprises an amount of vitamin K1 , vitamin K2, MGP or bioactive peptides derived from MGP effective to treat or prevent skin cancer or parakeratosis, to improve the skin integrity, the skin barrier function and/or to decrease skin ageing.

It will be described use of vitamin K is provided in the preparation of a food supplement, fortified food, nutraceutical, cosmeceutical, cosmetic, dermatological or pharmaceutical product for improving skin health and counteracting skin ageing, notably epidermal thinning, parakeratosis and loss of barrier function in healthy subjects and specifically in subjects and patients known to have a poor vitamin K status, but not patients receiving coumarin derivatives as anticoagulants.

It will further be described that said vitamin K-containing food supplements, nutraceutical, cosmeceutical or pharmaceutical products may further comprise medicines or other compounds for oral use, known to promote skin health or to treat a variety of skin diseases including (but not limited to): infections, psoriasis, keratosis, pseudoxanthoma elasticum, acne, eczema, herpes, shingles and vasculitis. Examples (not limiting) are: **antibiotics** such as erythromycin, tetracycline and dicloxacillin; **antifungal agents** such as ketoconazole, terbinafine and fluconazole; **antiviral agents** including valacyclovir, acyclovir, and famciclovir; **corticosteroids** such as prednisone; **immuno- suppressants** such as azothioprine and methotrexate; or **biological preparations** such as etanercept, adalimumab, infliximab, and ustekinumab.

It will further be described that said vitamin K-containing nutraceutical, cosmeceutical, cosmetic, dermatological or pharmaceutical products may further comprise medicines or other compounds for topical application, known to promote skin health or to treat a variety of skin diseases including (but not limited to): infections, psoriasis, keratosis, pseudoxanthoma elasticum, acne, eczema, herpes, shingles and vasculitis. Examples (not limiting) are: **antibacterials** such as mupirocin and clindamicin; **anti-inflammatory agents** like anthralin; **antifungal agents** such as terbinafine, clotrimazole, and ketoconazole; **agents for the treatment of acne** such as benzoyl peroxide, retinoids and salicylic acid; and **corticosteroids** (e.g. used for the treatment of eczema).

It will further be described that certain peptides derived from MGP (either human, mammals or vertebrates) are provided in the preparation of creams, ointments, sticks or lotions for improving skin health and counteracting skin ageing, notably epidermal thinning, parakeratosis and loss of barrier function in healthy subjects and specifically in subjects and patients known to have a poor vitamin K status, including patients receiving coumarin derivatives as anticoagulants. These bioactive peptides preferably contain the Gla-domain of MGP in whole or in part, and the glutamate residues may be carboxylated or uncarboxylated.

It will further be described that said bioactive peptides are used in cosmetic, dermatological or pharmaceutical products such as creams, ointments, sticks or lotions that may further comprise medicines or other compounds for topical application, known to promote skin health. Examples (not limiting) are: antibacterials such as mupirocin and clindamicin; anti-inflammatory agents like anthralin; antifungal agents such as terbinafine, clotrimazole, and ketoconazole; agents for the treatment of acne such as benzoyl peroxide, retinoids and salicylic acid; and corticosteroids (e.g. used for the treatment of eczema).

In an aspect of the present invention, vitamin K2 (phylloquinone and menaquinones) is used in the prevention or treatment of melanoma. Treatment with vitamin K is preferably combined with other forms of treatment such as surgical removal, radiation therapy or topical treatment (e.g. with fluorouracil).

The form of vitamin K used (in supplements) for improving skin health is menaquinone, possibly in combination with phylloquinone. Since, compared to phylloquinone, menaquinones have a higher efficacy, the active ingredient is preferably a menaquinone and most preferably it is selected from the long-chain menaquinones MK-7, MK-8, MK-9 or MK-10. Suitable daily doses of menaquinone to be used in the present invention are in the range between 5 and 5000 micrograms per day, preferably between 25 and 1000 micrograms per day, more preferably between 50 and 500 micrograms and most preferably between 100 and 250 micrograms per day. For patients with a known poor vitamin K status including (but not limited to) those with gastrointestinal disease, food malabsorption, bile obstruction, chronic kidney disease, coronary artery calcification, calcifylaxis, or peripheral artery disease, these ranges are twice as high.

These and other aspects of the present invention will be more fully outlined in the detailed description which follows.

### Brief description of the drawings

**Figure 1** shows the architecture of a normal, healthy skin, with two distinct layers: the dermis with fibroblasts as the dominant cell type and collagen and elastin fibrils providing strength and elasticity and the epidermis with its barrier function. The epidermis can be subdivided into several sub-layers (strata), and does not contain collagen or elastin.

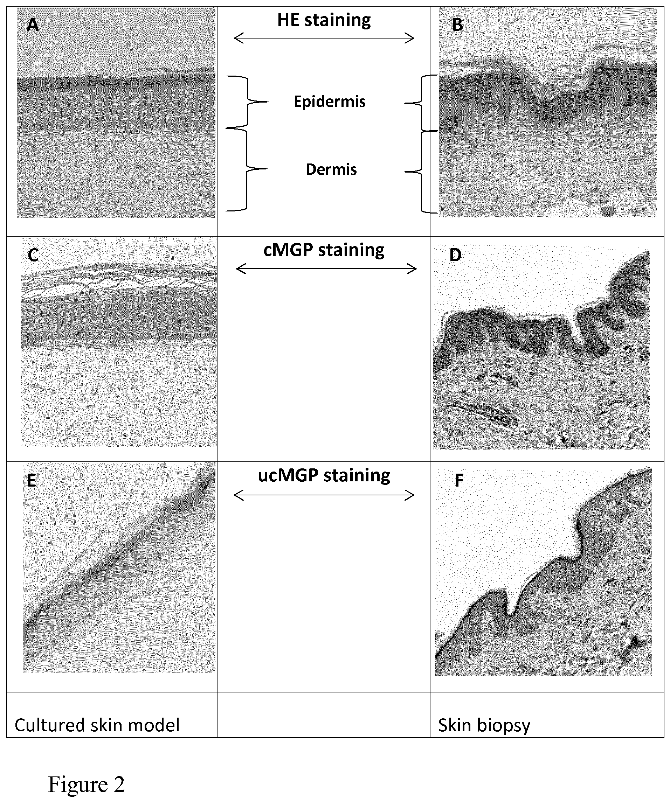

**Figure 2** shows different staining techniques on sections from cultured skin models and from in vivo biopsies. A and B: hematoxylin-eosin (HE) staining showing the dermis and the epidermis with strongest staining of the stratum granulosum. C and D: imunostaining using conformation-specific monoclonal antibodies against cMGP. Entire epidermis is positive for cMGP. E and F: immunostaining using conformation-specific monoclonal antibodies against ucMGP. Only stratum granulosum is strongly positive for ucMGP.

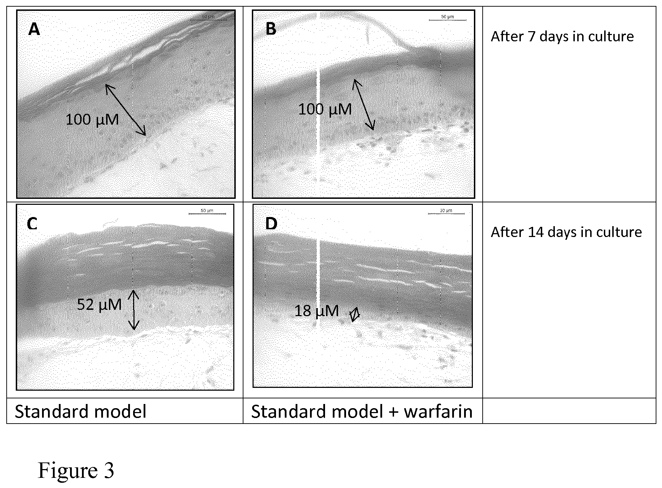

**Figure 3** shows the effect of warfarin-induced vitamin K insufficiency on epidermal cell proliferation in cultured skin models. The skin models were cultured for 7 days (A and B) and 14 days (C and D). The standard model shows a normal decline of epidermis: A and C; the warfarin-treated model shows a strong decline of epidermis: B and D. In most cases the epidermis is completely lost after 8-14 days of culture in warfarin- containing medium. The warfarin concentration (if added) in the culture medium was 10 µM. Staining: HE.

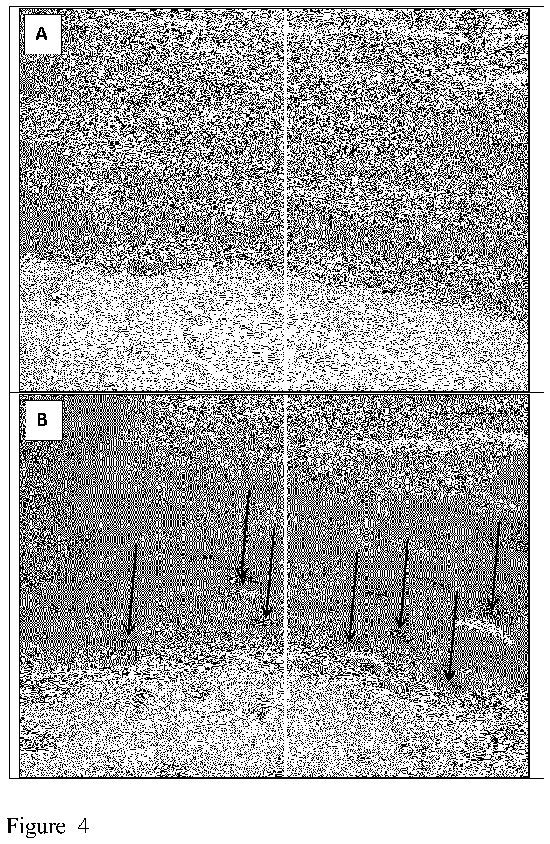

**Figure 4** shows parakeratosis as a result of warfarin-induced vitamin K insufficiency. This is a disturbed cell differentiation and migration of keratinocytes in the epidermis (arrows). A: standard skin model; B: warfarin-treated skin model. The warfarin concentration in figure 4B was 10 µM. Staining: HE.

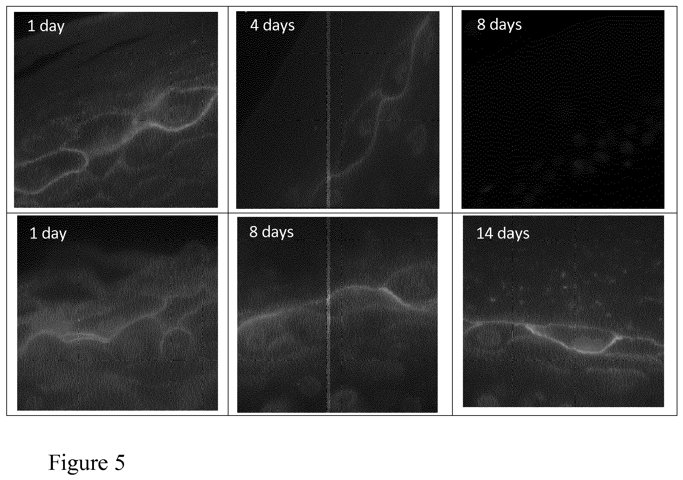

**Figure 5** shows the disappearance of ucMGP during warfarin treatment (three top panels), whereas it remains clearly present in the standard model (three bottom panels). The warfarin concentration (if added) was 10 µM. Staining of ucMGP was accomplished with fluorescently labeled monoclonal antibodies (in green). Note that pictures shown here represent 1 -4-8 days in warfarin culture and 1 -8-14 days in standard culture.

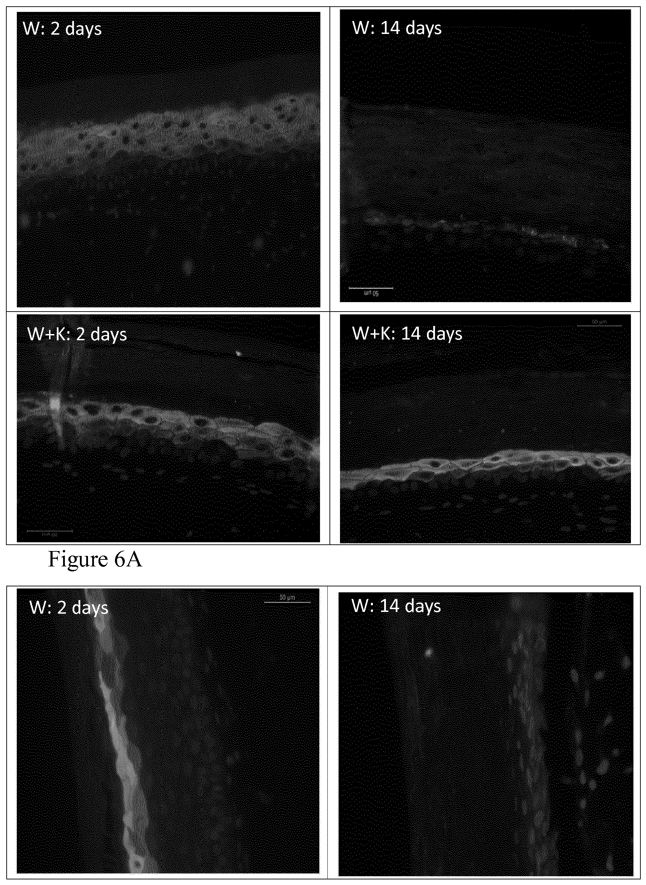

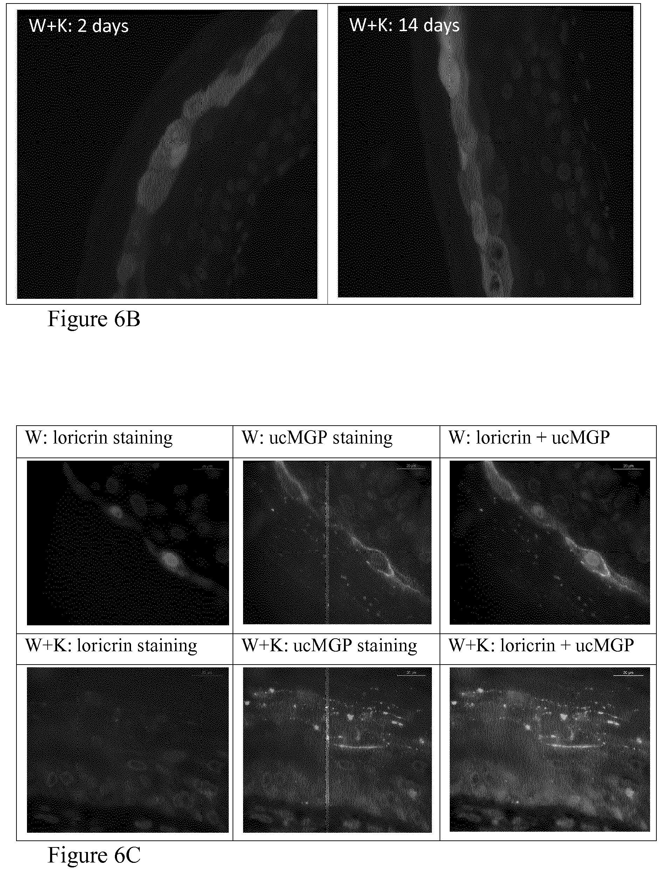

**Figure 6** shows that added vitamin K1 (50 µg/g of cetomacrogol cream) counteracts the warfarin-induced ageing of the epidermis. Vitamin K cream was applied once daily, the controls received cetomacrogol cream without additions.
   Figure 6A: Differentiation marker cytokeratin-10 measured in standard model + warfarin (W, top panels) and in standard model + warfarin + vitamin K (W+K, bottom panels). Staining with fluorescent-labeled antibody (green).
   Figure 6B: ucMGP disappears in standard model + warfarin (10 µM) and remains present in the model + warfarin + vitamin K. Staining with fluorescent-labeled antibody (green).
   Figure 6C: double staining with loricrin, a vitamin K-independent differentiation marker, (fluorescent probe = red) and ucMGP (fluorescent probe = green) showing that ucMGP coincides with differentiation marker (loricrin). Top panels: standard model with warfarin added to the culture medium, harvested after 4 days. Bottom panels: standard model with warfarin added to the culture medium and vitamin K in the cetomacrogol cream.

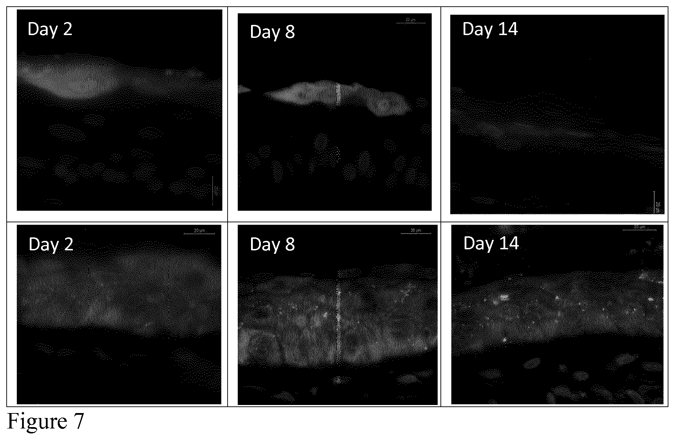

**Figure 7** shows the effect of warfarin on loricrin expression after 2, 8 and 14 days of culture in the absence (upper panel) and presence (lower panel) of ucMGP peptide 35-54 in a concentration of 200 microgram per gram of cream, and was applied once daily.

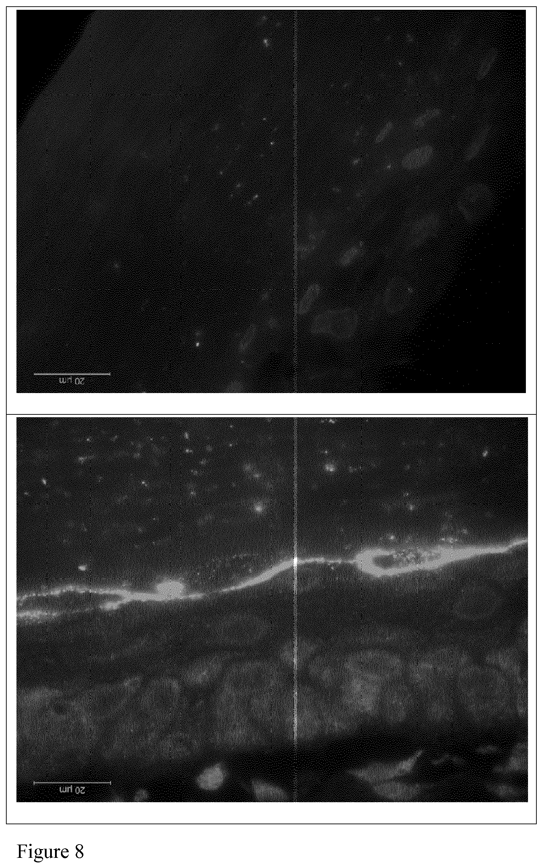

**Figure 8** shows that added ucMGP peptide (applied onto the skin model in a cream, 200 µg g) penetrates into the skin and accumulates in the stratum granulosum at the site where also endogenous ucMGP is found. Both panels after 14 days of culture. Top panel: Warfarin containing medium, no ucMGP peptide in cream. Bottom panel: Warfarin containing medium, cream containing ucMGP peptide 35-54. The added ucMGP peptide penetrates into the skin model and is visible at the stratum granulosum, precisely at the place where also endogenous ucMGP is found.

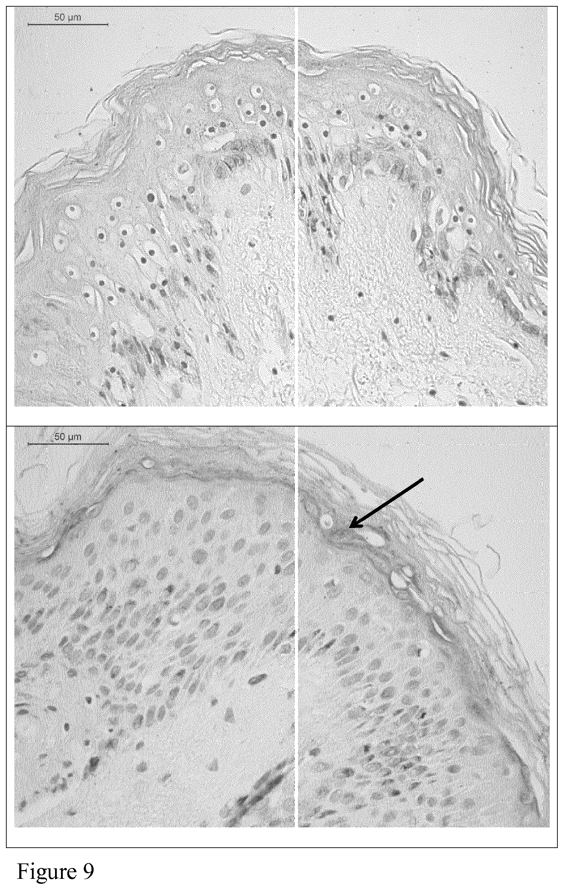

**Figure 9** shows the effect of vitamin K and warfarin in the full skin biopsy model supplied by Genoskin: epidermal thinning and disappearance of ucMGP. Top panel: model in the presence of warfarin and placebo cream. Bottom panel: model in the presence of warfarin + vitamin K2 cream (menaquinone-7, 5 µg g of cream). Staining for ucMGP (red-brown color, see arrow). Both panels show the model after 14 days of culture.

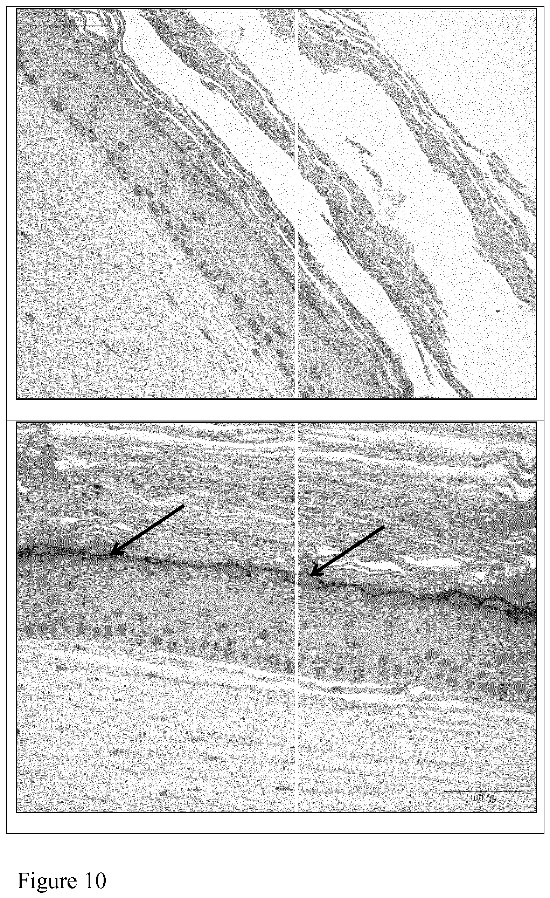

**Figure 10** shows similar effect in Biomimiq model. Top panel: model in the presence of warfarin and placebo cream. Bottom panel: model in the presence of warfarin + vitamin K2 cream (menaquinone-4, 10 µg g of cream). Staining for ucMGP (red-brown color, see arrow). Both panels show the model after 14 days of culture.

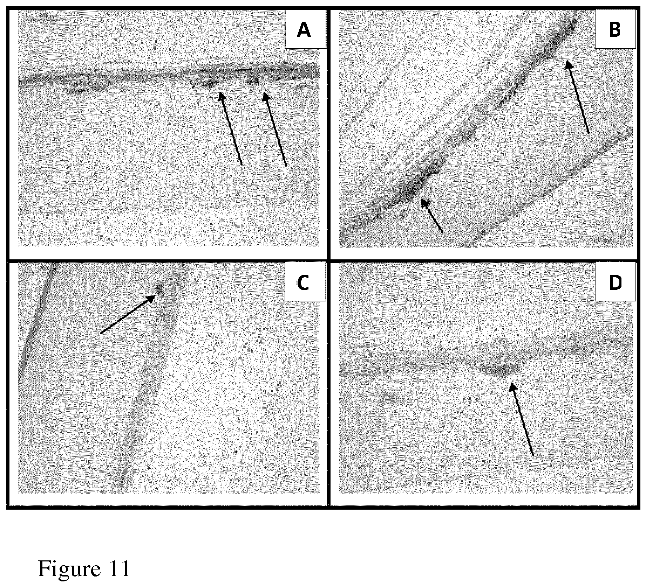

**Figure 11** shows the effect of vitamin K on melanoma cells. Panels A and B: growth of melanoma cells (red brown staining, arrows) using standard medium and cetomacrogol cream without further additions applied onto the skin surface at time points 10 (A) and 20 (B) days. Panels C and D: growth of melanoma cells using standard medium and cetomacrogol cream containing vitamin K2 (menaquinone-4, 10 µg g of cetomacrogol cream) onto the skin surface at time points 10 (C) and 20 (D) days of culture.

### Definitions and abbreviations

The term "vitamin K", as used herein, refers to both phylloquinone (also known as vitamin K-i) and menaquinone (also known as vitamin K2 ). Within the group of vitamin K2 , special reference is made to menaquinone-4 (MK-4) and the long-chain menaquinones (MK-7, MK-8 and MK-9), in particular menaquinone-7 (MK-7). It is generally accepted that the methylated naphthoquinone group, which all K vitamins have in common, is the functional group so that the mechanism of action is similar for all K vitamins. Differences may be expected, however, with respect to intestinal absorption, transport, tissue distribution, and bioavailability.

As used herein, the terms "effective amount" and "therapeutically effective amount" are interchangeable and refer to an amount that results in bringing the desired effect.

As used herein, the term "vitamin K status" refers to the extent to which various circulating Gla-proteins have been carboxylated. Poor vitamin K status means that the dietary vitamin K intake is insufficient to ensure complete Gla-protein carboxylation. Both ucOC and dp-ucMGP are well recognized as sensitive markers for poor vitamin K status.

In general a distinction is made between hepatic vitamin K status (carboxylation of coagulation factors) and extra-hepatic vitamin K status (carboxylation of Gla-proteins not synthesized in the liver). The liver produces the vitamin K-dependent blood coagulation factors. Insufficient hepatic vitamin K status is extremely rare; therefore, the clotting factors are no sensitive markers for vitamin K status. Unless stated otherwise, the term vitamin K status as used herein refers to both hepatic vitamin K status and extra- hepatic vitamin K status.

In the present patent specification a person's vitamin K status will be regarded as poor when the circulating levels of uncarboxylated extra-hepatic Gla-proteins MGP (measured as dp-ucMGP) and/or osteocalcin (as measured as ucOC) are above the upper normal level in healthy adults.

The term "Gla" stands for γ-carboxyglutamic acid, an unusual amino acid which is formed post-translationally by vitamin K action.

MGP stands for Matrix Gla-protein, one of the 17 vitamin K-dependent proteins presently known. Mature MGP contains five Gla-residues, four of which are located in what is called the "Gla-domain", i.e. the amino acid sequence 35-54 in human MGP. Likewise, ucMGP stands for uncarboxylated MGP and cMGP for carboxylated MGP. Unless stated otherwise, the term "MGP" as used herein refers to any form of Matrix Gla Protein, either in its carboxylated or uncarboxylated form and either in its phosphorylated or non- phosphorylated form, and mixtures thereof.

As used herein, the term "MGP-derived peptide" refers to a sequence with a length between 5 and 54 amino acid residues which is substantially homologous, i.e. having at least 40%, preferably at least 60% and most preferably at least 80% sequence similarity to the corresponding form of human MGP.

GGCX stands for γ-glutamate carboxylase, the vitamin K-dependent enzyme catalysing the posttranslational carboxylation of all Gla-containing proteins.

The epidermis is composed of the outer layer of the two main layers that make up the skin, which main function is that of forming a barrier against external influences. The epidermis is composed of proliferating basal and differentiated suprabasal keratinocytes, and acts as the body's major barrier against an inhospitable environment by preventing pathogens and hazardous compounds from entering, making the skin a natural barrier to infections and harmful agents. The deepest part of the epidermis also contains melanocytes, which produce melanin giving the skin its colour.

The dermis is a layer of inner layer of the two main layers that make up the skin and is mainly composed of connective tissue that provides the skin its elasticity and strength. The dermis is tightly connected to the epidermis through a basement membrane. Structural components of the dermis are collagen and elastic fibrils and also contains hair follicles, sweat glands, lymphatic vessels and blood vessels. These blood vessels provide nourishment and waste removal for both dermal and epidermal cells.

Parakeratosis is characterized by the retention of nuclei in the stratum corneum. In the skin this leads to the abnormal replacement of annular squames with nucleated cells. Parakeratosis is associated with the thinning of the stratum granulosum and is usually seen in diseases associated with increased cell turnover, whether inflammatory or neoplastic.

Loricrin and cytokeratin-10 are markers for epidermal differentiation.

Warfarin is known as a "vitamin K antagonist" and is a direct inhibitor of the enzyme vitamin K-epoxide reductase (VKOR), which is responsible for the recycling of vitamin K into the active form vitamin K hydroquinone. Once VKOR is inhibited, vitamin K can only be used once, resulting in a 1000 - 2000 fold increased vitamin K requirement.

For the purposes of this specification and the appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters set forth, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

It is noted that, as used in this specification and the appended claims, the singular forms "a," "an," and "the," include plural referents unless expressly and unequivocally limited to one referent. Thus, for example, reference to "a monomer" includes two or more monomers.

### Detailed description of the invention

The invention is based on the identification of both cMGP and ucMGP in the epidermal layer of the skin, and on the surprising discovery that poor vitamin K status, herein induced by the vitamin K antagonist, results in accelerated skin ageing, quick epidermal thinning, parakeratosis, and concomitant disappearance of MGP, notably ucMGP. Since MGP is a calcium-binding protein, this will also affect the calcium gradient known to be present in the stratum granulosum, which forms the main barrier in the skin against foreign influences.

Surprisingly, we have found that warfarin-induced vitamin K insufficiency is a strong risk factor for skin ageing, which manifests itself in accelerated thinning of the epidermis and parakeratosis. In the skin, parakeratosis leads to the abnormal replacement of annular squames with nucleated cells and is associated with the thinning or loss of the stratum granulosum; it is often seen in diseases of increased cell turnover. Remarkably carboxylated MGP (cMGP) is present in the entire epidermis, whereas uncarboxylated MGP (ucMGP) is mainly located in the stratum granulosum.

In contrast to the general knowledge, ucMGP disappeared during warfarin treatment. The association between ucMGP and skin health suggests that - in analogy of ucOC - also ucMGP may originate from cMGP by decarboxylation and act as a hormone in metabolic processes other than its primary role (calcification inhibition).

It was also found that vitamin K counteracts the effect of warfarin, i.e. it blocks the epidermis thinning and parakeratosis, and - surprisingly - it also results in an increase of ucMGP production.

Synthetic peptides analogous to the 35-54 domain (generally known as the "Gla-domain") of human MGP turned out to completely abolish the warfarin-induced skin ageing in cultured skin systems. Similar effects were observed with larger peptides containing the MGP Gla-domain, irrespective whether they were carboxylated or uncarboxylated. Moreover, these peptides were demonstrated to penetrate into the skin and locate at the same site as endogenous MGP molecules are found. Collectively these MGP-derived peptides are referred to herein as bioactive peptides.

Our experiments clearly demonstrate that both vitamin K and MGP-derived bioactive peptides are important for skin health, more in particular epidermal integrity, vitality and barrier function. Since vitamin K is light-sensitive, oral supplementation may be preferred over topic application. However, this is problematic during oral anticoagulation. Therefore, an alternative is to apply said bioactive MGP-derived peptides in creams, ointments, sticks or lotions.

These observations were made in cultured healthy skin, but were even more apparent in skin cultures grown in the presence of vitamin K antagonists. Therefore our findings are even more important for patients known to have a poor vitamin K status including but not limited to chronic kidney disease patients, pregnant women, cigarette smokers, patients with Crohn's disease, bile obstruction or renal transplant recipients and patients receiving coumarin anticoagulants. Our findings are also important for wound healing, for instance after a cut, a trauma, rash, fissure or after burning.

Surprisingly, we also found another activity of K vitamins, namely antiproliferative activity in a skin model for melanoma (skin cancer). Vitamin K suppresses melanoma cells and retards their growth. Whereas all forms of vitamin K were capable of inhibiting melanoma cell growth, MGP-derived peptides were devoid of such activity. This demonstrates that the mechanism of action in tumour suppression is different and independent of MGP. Without wishing to be bound to any theory we believe that the mechanism underlying this activity may either be based on cell growth regulation via the vitamin K-dependent protein Gas6 or on directly affecting transcription via the SXR nuclear receptor. The effective amount of vitamin K in the topical preparations is in the range between 0.5 and 100 µg g of carrier material such as cream, ointment, stick or lotion, more preferably between 1 and 20 µg g and most preferably between 2 and 10 µg g. In subjects receiving oral anticoagulant medication the effective concentration of vitamin K will be in the upper range as defined herein.

Thus, in one aspect the present invention broadly relates to a new and completely unexpected application of vitamin K2, wherein vitamin K2 is used to treat or prevent skin cancer, counteract epidermal ageing, improve epidermal health and survival of the keratin ocytes, melanocytes, Langerhans cells and Merkel cells which are known to be present in the healthy epidermis. Vitamin K will also help improve the barrier function of the stratum granulosum and thus contribute to skin health in general. Vitamin K exerts this effect after topical application in creams, ointments, sticks and lotions, but because its light sensitivity, the efficacy will be limited unless used in night creams or in combination with sun blockers. Therefore oral applications in the form of pharmaceutical, cosmeceutical or nutraceutical food supplements or functional foods are preferred, except in case of patients on oral anticoagulant medication such as warfarin, acenocoumarol and phenprocoumon. These drugs are antagonists of vitamin K and oral vitamin K preparations are contra- indicated in these patients. For this group topical application of vitamin K is the preferred route.

In preparations for topical use, the effective concentration of vitamin K in performing the invention will be in the range between 0.5 and 100 µg g of carrier material such as cream, ointment, stick or lotion, more preferably between 1 and 20 µg g and most preferably between 2 and 10 µg g. In subjects receiving oral anticoagulant medication the effective concentration of vitamin K will be in the upper range as defined herein.

In preparations for oral use the dose of vitamin K effective in performing the invention is not restricted but may be established individually under guidance of the extent to which circulating dp-ucMGP, ucOC or other uncarboxylated Gla-proteins are decreased as a result of the treatment. Current AI values (i.e. Adequate Intakes, as determined by the Institute of Medicine) are 120 µg for healthy men and 90 µg for healthy women. Where national legislation permits, it may be advisable to provide dosage ranges between 5 µg day and 5 mg/day, preferably between 25 µg day and 1000 µg day, more preferably between 50 µg day and 500 µg day, and most preferably between 100 and 250 µg day. The duration of the intervention is preferably lifelong. In terms of body weight, daily dosages may range between 0.05 and 50 µg kg body weight, preferably between 0.25 and 10 µ9 I<9 body weight, more preferably between 0.5 and 5 µ9 I<9 body weight and most preferably between 1 and 2.5 µ9 I<9 body weight.

Vitamin D may be included together with vitamin K in the compositions described in this invention since it is well known that both osteocalcin and MGP have a vitamin D-responsive element in their promoter sequence and that expression of these proteins may thus be stimulated by vitamin D. Any form of natural or synthetic vitamin D may be employed, including vitamin D-i , vitamin D2 (calciferol), vitamin D3 (cholecalciferol) and vitamin D analogues (e.g. alfacalcidol, dihydrotachysterol, calcitriol). Natural sources of vitamin D, include saltwater fish, organ meats, fish-liver oils and egg yolk. Suitable dosages of vitamin D are 2 to 50 µg day, preferably 5 to 20 µg day, and most preferably about 7 to 10 µg day.

For use in the present invention, phylloquinone, MK-4 and the long-chain menaquinones MK-7, MK-8 and MK-9 are preferred, and MK-7 is particularly preferred. Sources of vitamin K which can be used according to the present invention include the following: phylloquinone from natural sources, such as vegetable extracts, fats and oils, synthetic phylloquinone, different forms of vitamin K2 : synthetic MK-4, MK-5, MK-6, MK-7, MK-8, MK-9, MK-10, MK-1 1 , MK-12 and MK-13, natto (food prepared from fermented soybean, rich in MK-7), natto extracts, and other fermented foods or dairy products.

Vitamin K-enriched nutritional products can be manufactured to provide the daily requirements of vitamin K. For example, vitamin K can be added to food products, such as, for example, meal replacers, ice cream, sauces, dressings, spreads, bars, sweets, snacks, cereals, beverages, etc. by methods as described in EP 1 153548 and US 8,354,129, the entire disclosure of which is incorporated by reference herein. Also, vitamin K can be used in food supplements such as multivitamins, tablets, capsules, sachets, and other forms.

Vitamin K is conventionally provided in the form of tablets or capsules, i.e. in a pharmaceutical or dietary supplement format. For pharmaceutical preparations or dietary supplements the vitamin K may be compounded with pharmaceutically acceptable carriers, excipients or diluents in the forms of pills, tablets (coated or uncoated), hard or soft capsules, dragees, lozenges, oral solutions, suspensions and dispersions, syrups or sterile parenteral preparations. Suitable excipients include inert diluents, such as calcium carbonate, sodium carbonate, lactose, calcium phosphate, sodium phosphate; granulating and disintegrating agents, such as cornstarch or alginic acid; binding agents, such as starch, gelatin or acacia; effervescents; and lubricating agents, such as magnesium stearate, stearic acid or talc. It is also possible to deliver or administer Vitamin K (optionally together with vitamin D) in a fortified food or beverage product. Preferred nutritional product formats include: juice drinks, dairy drinks, powdered drinks, sports drinks, mineral water, soy beverages, hot chocolate, malt drinks, biscuits, bread, crackers, confectioneries, chocolate, chewing-gum, margarines, spreads, yoghurts, breakfast cereals, snack bars, meal replacements, protein powders, desserts, and medical nutrition-tube feeds and nutritional supplements.

The effects of vitamin K are probably exerted via the vitamin K-dependent protein MGP. Tissue MGP is synthesized locally and vitamin K is a cofactor in this process. Since MGP was identified in the epidermal layers, the most plausible mechanism underlying vitamin K action is via MGP. This links the above-mentioned effects of vitamin K and the surprising observation that MGP and MGP-derived peptides exhibit essentially the same effects on epidermal health.

In a further aspect, the present invention also broadly relates to a new and completely unexpected application of MGP and MGP-related peptides, wherein said compounds are used to counteract epidermal ageing, improve epidermal health and survival of the keratin ocytes, melanocytes, Langerhans cells and Merkel cells which are known to be present in the healthy epidermis. MGP and MGP-related peptides (both in their carboxylated and uncarboxylated form) will also help improve the barrier function of the stratum granulosum and thus contribute to skin health in general.

These MGP and MGP-related peptides exert their effect preferably after topical application such as in creams, ointments, sticks and lotions. In contrast to vitamin K they cannot be used as such in preparations for oral use because they are readily hydrolysed in the digestive tract. Therefore, applications in cosmetic products or products for dermatological care are preferred. An advantage is that these products can also be used for patients on oral anticoagulant medication such as warfarin, acenocoumarol and phenprocoumon.

The synthetic bioactive peptides used (in topical preparations such as creams, ointments, sticks and lotions) for improving skin health are selected from the amino acid sequence 1 -54 of human MGP: Tyr-Glu-Ser-His-Glu-Ser-Met-Glu-Ser-Tyr-Glu-Leu-Asn- Pro-Phe-Ile-Asn-Arg-Arg-Asn-Ala-Asn-Thr-Phe-Ile-Ser-Pro-Gln-Gln-Arg-Trp-Arg-Ala-Lys- Val-Gln-Glu-Arg-Ile-Arg-Glu-Arg-Ser-Lys-Pro-Val-His-Glu-Leu-Asn-Arg-Glu-Ala-Cys, in which the amino acid residues on positions 2, 37, 41 , 48 and 52 may be either in their uncarboxylated (glutamate) conformation or in their carboxylated (γ-carboxyglutamate) conformation. Smaller bioactive peptides should preferably include the entire or part of the MGP Gla-domain, i.e. sequence 35-54: Val-Gln-Glu-Arg-Ile-Arg-Glu-Arg-Ser-Lys-Pro-Val- His-Glu-Leu-Asn-Arg-Glu-Ala, in which the four Glu residues may be either carboxylated or uncarboxylated. Where these amino acid sequences contain serine residues, these residues may be either in the phosphorylated or in the non-phosphorylated form. For reasons of cost, the uncarboxylated (Glu) conformation is preferred. Also chimeric constructs in which these sequences are linked to other peptides, proteins, sugars, lipids or phospholipids may be used as clinically effective products.

The preferred effective peptide concentration in the topical preparations is in the range between 1 µg g and 1000 µg g of cream, more preferred between 5 µg g and 200 µg g of cream, and most preferred in the range of 10 µg g and 100 µg g of carrier material such as cream, ointment, stick or lotion.

Having now generally described the invention, the same may be more readily understood through the following reference to the experimental part including the examples, which are provided by way of illustration and are not intended to limit the present invention unless specified.

### Experimental

### Methods

In this invention we describe a number of new and unexpected phenomena in skin and skin models. Except the melanoma model (which was exclusively from Mattek) all findings were reproduced in models from four different suppliers: Mattek, Genoskin, Straticell and Biomimiq and essentially the same outcomes were obtained. For sake of uniformity, only the results with Mattek systems are shown in the Figures 2 through 8. After receipt of the skin models, they were grown in the medium provided by the supplier at 37°C for periods between 1 and 14 days (as indicated); in case warfarin was added to the system, this was via the culture medium in a concentration of 10 µM. In case vitamin K or MGP or MGP-derived peptides were added to the system, this was via application in cetomacrogol cream using an application stick; the concentrations are given in the detailed description of the experiments. After termination of the experiments, the cultured skin specimen were harvested, embedded in paraffin according to standard procedures and prepared for immunohistochemistry. Staining was performed with HE, with monoclonal antibodies against: uncarboxylated MGP (ucMGP), carboxylated MGP (cMGP), osteocalcin, Gas6, loricrin, cytokeratin-10, and polyclonal antibodies against MGP (C-terminal domain) and GRP (C-terminal domain) using either HRP or fluorescent probes (as indicated). These antibodies were used in dilutions ranging from 0.1 to 10 µg mL (as empirically determined to be optimal) in phosphate-buffered saline according to standard techniques. In the case of melanoma cells, staining was performed using polyclonal anti- Si 00 antibodies from Dako, where S100 proteins were used as tumor marker.

### Results

In a first set of experiments we have demonstrated the occurrence of both carboxylated and uncarboxylated MGP in healthy skin and in cultured skin models (Figure 2). The location of ucMGP and cMGP was different, however whereas cMGP was located all over the epidermis, ucMGP was abundantly found associated with a narrow band known as the stratum granulosum and suggests different biological activities of both forms. Remarkably, no MGP (any form) was found in the dermis.

The occurrence of both carboxylated and uncarboxylated MGP in the skin cultures indicates a delicate balance regulated by vitamin K and stimulated us to investigate the effect of vitamin K insufficiency on the cultured skin models. Since the ready-made culture medium contains significant amounts of vitamin K, it is difficult to design experiments in the absence of vitamin K. Therefore we added a vitamin K antagonist (warfarin, supplied via the culture medium) to the systems and in this way we created an artificial form of vitamin K deficiency. Surprisingly, we found three effects of warfarin on skin and skin ageing: (i) warfarin accelerates the thinning and disappearance of the epidermis (cell proliferation, see Figure 3); (ii) warfarin induces parakeratosis (cell differentiation, see figure 4); and (iii) warfarin induces a decrease in ucMGP (see Figure 5). Notably the latter observation is counter-intuitive and not consistent with present knowledge on warfarin action, but the effect was invariably seen in all skin models and in repeated experiments.

In a number of subsequent experiments, examples of which will be detailed below, we have demonstrated that the effect of warfarin was not based on cell toxicity, but on the antagonism of vitamin K action. Moreover, we show that certain peptides homologous or with a certain degree of sequence similarity to human MGP, stimulate epidermal growth and counteract parakeratosis in the warfarin-induced ageing of cultured skin models. Similar effects were found in cultured human skin biopsies as provided by Genoskin.

In a final experiment (see Figure 1 1 ) we also show that various forms of vitamin K inhibit or delay the growth of skin cancer, notably the most dangerous form melanoma. Remarkably, the abovementioned MGP-derived peptides had no effect in this model, which demonstrates that the underlying mechanism is different from that shown in Figures 2-10. The effect on melanoma cells may be exerted either via a direct effect of vitamin K (probably via the nuclear SXR receptor) or via the vitamin K-dependent protein Gas6.

### Conclusions

1 . Poor vitamin K status is an independent risk factor for epidermal skin health and ageing, and may also adversely affect its barrier function. Since MGP is a strong calcium binding protein, its function in the stratum granulosum may be the maintenance of the local calcium gradient, which helps to protect the skin from adverse outside influences.
2. Both vitamin K (administered either orally or topically) and MGP-derived peptides (applied topically) are capable to counteract the negative effects of vitamin K insufficiency in the skin.
3. Poor vitamin K status of the skin is also an independent risk factor for skin cancer proliferation. Both oral administration and topical application of vitamin K may support other therapies of skin cancer.

### Example 1 (see Figure 6)

Here we demonstrated that vitamin K1 is capable of counteracting the effects of warfarin. In these experiments warfarin was added to the system via the culture medium, whereas vitamin K1 was applied in a cream (cetomacrogol). The concentration of warfarin was 10 µM, the concentration of vitamin K1 was 50 pmol/g of cream. Both the culture medium and the cream were refreshed daily, and the cultures were continued for 2 weeks. It turned out that vitamin K1 counteracted the warfarin-induced decrease of epidermal proliferation and differentiation (see example in Figure 6A) whereas at the same time it resulted in an increase of ucMGP accumulation in the entire epidermis, although most of ucMGP accumulated in association with the stratum granulosum (Figure 6B). In Figure 6C we demonstrated that ucMGP coincides with loricrin, a marker for epidermal cell differentiation which is known to be mainly present in the stratum granulosum. These data prove that indeed the stratum granulosum is the place of ucMGP accrual.

### Example 2 (see Figures 7 and 8)

Subsequently, a MGP-derived peptide (prepared by Pepscan) homologous to the Gla-domain (uncarboxylated amino acid sequence 35-54) was added in the warfarin-treated model. The peptide were applied once daily via a cetomacrogol cream in a concentration of 200 µg/g of cream. It resulted that not only the 35-54 peptide counteracted the effects of warfarin (see Figure 7), but also these effects were observed with both the carboxylated and uncarboxylated conformations. Similar results were obtained with longer peptides homologous to the amino acid sequences 1 -54 of human MGP. Remarkably, it was observed that in case cMGP peptides were used, also ucMGP was detected in the skin model. This demonstrates that cMGP was decarboxylated into ucMGP, which is strongly suggestive for a biological function of ucMGP. The location of the added MGP peptides was similar to that where the native MGP was found to be present, i.e. entire epidermis for cMGP peptides and strong accumulation of ucMGP in the stratum granulosum (see Figure 8).

### Example 3 (see Figure 9)

The warfarin-induced thinning and disappearance of the epidermal layer and the concomitant disappearance of ucMGP was also demonstrated in full skin biopsies obtained from Genoskin, images shown were obtained after 14 days of culture (Figure 9) in warfarin-containing medium without (top) and with vitamin K (menaquinone-7, bottom). Also in this model the effects of warfarin were clearly visible and counteracted by vitamin K. Moreover, this examples clearly demonstrates that besides vitamin K-i also MK-7 is capable of counteracting the effects of warfarin-induced vitamin K deficiency.

### Example 4 (see Figure 10)

The warfarin-induced thinning and disappearance of the epidermal layer and the concomitant disappearance of ucMGP was also demonstrated in the skin model supplied by Biomimiq, images shown were obtained after 14 days of culture (Figure 10) in warfarin-containing medium without (top) and with vitamin K (menaquinone-4, bottom). Also in this model the effects of warfarin were clearly visible and counteracted by vitamin K. Moreover, this examples clearly demonstrates that besides vitamin K-i and MK-7, also MK-4 is capable of counteracting the effects of warfarin-induced vitamin K deficiency.

### Example 5 (see Figure 1 1 )

In the melanoma skin cancer model provided by Mattek, a regular growth of melanoma cells was observed under standard conditions, and empty carrier (cetomacrogol cream) applied on a daily basis. Here are given staining after 10 and 20 days of growth (Figures 1 1 A and B). A progressive accumulation of tumor cells can be seen. When vitamin K was added to the cetomacragol cream (10 g/g) the growth of melanoma cells was markedly retarded (Figures 1 1 C and D) but not completely blocked. In the figure shown menaquinone-4 (MK-4) was the form of vitamin K2 used, but essentially the same data were obtained for phylloquinone (vitamin K1 ) and MK-7.

### Example 6

Vitamin K was mixed in cetomacrogol cream at a dose of 1 µg g of cream and stored for 1 week at room temperature. Three formulations were prepared: vitamin K1 (phylloquinone), MK-4 (menaquinone-4) and MK-7 (menaquinone-7). All K vitamins were obtained as pure compounds from Sigma-Aldrich, Missouri (K1 and MK-4) and from Cato Research Chemicals, Oregon (MK-7). These preparations were analyzed for vitamin K content using standard extraction and HPLC techniques, and the recoveries were: 98% (K1 ), 96% (MK-4) and 102% (MK-7). This demonstrates that the vitamin K in creams is stable and can be kept at room temperature without significant losses.

### Example 7

Bioactive uncarboxylated peptide MGP1 -54 was added to cetomacrogol cream at a dose of 200 µg g of cream and stored for 1 week at room temperature. The peptide was obtained from Pepscan (Lelystad, The Netherlands). At baseline and after one week of storage, 10 mL of phosphate-buffered saline and 0.5 grams of lipase were added per gram of cream and incubated for 1 h at 37°C. Subsequently, the peptide was isolated by immunoprecipitation. Western blot analysis showed one clear band with the expected molecular weight of 6 kD and similar intensity at baseline and after one week of storage. This demonstrates that the bioactive peptide-containing creams are stable with no detectable loss during storage.

### Formulation of products and dosages

If protected from light, all forms of vitamin K are stable at room temperature with no significant loss after 2 years of storage. The route of administration is either systemic (e.g. orally or parenterally), or topical (e.g. in creams, ointments, sticks and lotions). Systemic administration is preferably performed in the form of capsules, tablets or fortified foods. Since it is a fat-soluble vitamin, it is preferably formulated in oil, for instance fish oil or sunflower seed oil and manufactured in the form of soft or hard gelatin capsules. Vitamin K can also be formulated in tablets comprising pharmaceutically acceptable inactive ingredients such as starch, cellulose, magnesium stearate or mixtures thereof. These products can be prepared by conventional manners known in the art and typically have a weight between 100 milligrams and 1 gram. Moreover, it is well known that through nano-encapsulation fat-soluble vitamins including vitamin K can be prepared in a water- soluble form. The effective dose of vitamin K for oral use per capsule or tablet is typically between 5 and 5000 micrograms, preferably between 25 and 1000 micrograms, more preferably between 50 and 500 micrograms and most preferably between 50 and 250 micrograms. The effective dose of vitamin K for topical use in creams, ointments, sticks and lotions is typically between 0.5 and 100 micrograms per gram of cream, ointment, stick or lotion, more preferably between 1 and 20 micrograms per gram, and most preferably between 2 and 10 micrograms per gram.

For the preparation and formulation of products for topical application of bioactive peptides, it should be noted that these peptides should have a certain sequence similarity with the amino acid sequence of certain human MGP domains, but that the sequence similarity does not need to be 100%. For the sake of clarity a bioactive peptide herein is defined as a peptide having a sequence similarity of at least 40% with a certain domain in human MGP. Preferred bioactive peptides are prepared via common peptide synthesis and in the uncarboxylated (i.e. all glutamate residues as GLU) form because this is the most cost-effective form; however, also fully or partially carboxylated peptides (i.e. one or more glutamate residues occur as GLA) may be used. Effective concentrations may depend on the polypeptide length, but effective doses will range between 1 and 1000 µg g of cream, ointment, stick or lotion, more preferably between 5 and 200 µg g and most preferably between 10 and 100 µg g of cream, ointment, stick or lotion. Topical application should occur at least once daily, preferably 2-3 times daily. Since the bioactive peptides are not light sensitive they may be used in products that are exposed to daylight after application.

## Claims

1. A pharmaceutical composition for use in the treatment or prevention of melanoma in a human subject, wherein the composition comprises a therapeutically effective amount of vitamin K2.

2. The pharmaceutical composition for use according to claim 1, wherein said composition further comprises phylloquinone (vitamin K1).

3. The pharmaceutical composition for use according to claim 1 or 2, wherein said vitamin K2 comprises one of the long-chain menaquinones MK-7, MK-8, MK-9, MK-10, or a combination thereof.

4. The pharmaceutical composition for use according to any one of claims 1 to 3, wherein said vitamin K2 is administered in addition to the normal dietary intake of vitamin K as a functional food, a food supplement or a pharmaceutical preparation.

5. The pharmaceutical composition for use according to any one of claims 1 to 4, wherein said vitamin K2 is administered orally in a dose range between 5 and 5000 µg/day, preferably between 25 and 1000 µg/day, more preferably between 50 and 500 µg/day and most preferably between 100 and 250 µg/day.

6. The pharmaceutical composition for use according to any one of claims 1 to 5, wherein vitamin K2 is administered topically, in particular in a cream, ointment, stick or lotion, preferably in a concentration range between 0.5 and 100 µg/g of cream, ointment or lotion, morepreferably between 1 and 20 µg/g and most preferably between 2 and 10 µg/g of cream, ointment or lotion.

7. The pharmaceutical composition for use according to any one of claims 1 to 6, wherein said subject has a poor vitamin K status, preferably the subject has a poor hepatic vitamin K status and/or a poor extra-hepatic vitamin K status.

8. The pharmaceutical composition for use according to any one of claims 1 to 7, wherein said vitamin K2 is for administration:
a. in combination with at least one other food or food supplement known to improveskin health, in particular antioxidants or omega-3 fatty acids, or
b. in combination with at least a pharmaceutically, cosmeceutically or cosmetically active product, in particular hyaluronic acid, dimethylaminoethanol, or an antibiotic, corticosteroid or antifungal medicine.

9. The pharmaceutical composition for use according to any one of claims 1 to 8, wherein said composition further comprises vitamin D, preferably the dosages of vitamin D are 2 to 50 µg/day, more preferably 5 to 20 µg/day, and most preferably 7 to 10 µg/day.

## Patentansprüche

1. Pharmazeutische Zusammensetzung zur Verwendung bei der Behandlung oder Vorbeugung von Melanomen bei einem menschlichen Subjekt, wobei die Zusammensetzung eine therapeutisch wirksame Menge an Vitamin K2 umfasst.

2. Pharmazeutische Zusammensetzung zur Verwendung nach Anspruch 1, wobei die Zusammensetzung ferner Phyllochinon (Vitamin K1) umfasst.

3. Pharmazeutische Zusammensetzung zur Verwendung nach Anspruch 1 oder 2, wobei das Vitamin K2 eines der langkettigen Menachinone MK-7, MK-8, MK-9, MK-10 oder eine Kombination davon umfasst.

4. Pharmazeutische Zusammensetzung zur Verwendung nach einem der Ansprüche 1 bis 3, wobei das Vitamin K2 zusätzlich zu der normalen Nahrungsaufnahme von Vitamin K als funktionelles Lebensmittel, Nahrungsergänzungsmittel oder pharmazeutische Zubereitung verabreicht wird.

5. Pharmazeutische Zusammensetzung zur Verwendung nach einem der Ansprüche 1 bis 4, wobei das Vitamin K2 oral in einer Dosierung in dem Bereich von 5 bis 5000 µg/Tag, bevorzugt zwischen 25 und 1000 µg/Tag, noch bevorzugter zwischen 50 und 500 µg/Tag und am meisten bevorzugt zwischen 100 und 250 µg/Tag verabreicht wird.

6. Pharmazeutische Zusammensetzung zur Verwendung nach einem der Ansprüche 1 bis 5, wobei Vitamin K2 topisch verabreicht wird, insbesondere in einer Creme, Salbe, einem Stick oder einer Lotion, bevorzugt in einer Konzentration in dem Bereich zwischen 0,5 und 100 µg/g Creme, Salbe oder Lotion, noch bevorzugter zwischen 1 und 20 µg/g und am meisten bevorzugt zwischen 2 und 10 µg/g Creme, Salbe oder Lotion.

7. Pharmazeutische Zusammensetzung zur Verwendung nach einem der Ansprüche 1 bis 6, wobei das Subjekt einen schlechten Vitamin-K-Status aufweist, bevorzugt weist das Subjekt einen schlechten hepatischen Vitamin-K-Status und/oder einen schlechten extrahepatischen Vitamin-K-Status auf.

8. Pharmazeutische Zusammensetzung zur Verwendung nach einem der Ansprüche 1 bis 7, wobei das Vitamin K2 zur Verabreichung bestimmt ist:
a. in Kombination mit mindestens einem anderen Lebensmittel oder Nahrungsergänzungsmittel, das bekanntermaßen die Hautgesundheit verbessert, insbesondere Antioxidantien oder Omega-3-Fettsäuren, oder
b. in Kombination mit mindestens einem pharmazeutisch, kosmezeutisch oder kosmetisch wirksamen Produkt, insbesondere Hyaluronsäure, Dimethylaminoethanol oder einem Antibiotikum, Kortikosteroid oder Antimykotikum.

9. Pharmazeutische Zusammensetzung zur Verwendung nach einem der Ansprüche 1 bis 8, wobei die Zusammensetzung ferner Vitamin D umfasst, wobei die Dosierungen von Vitamin D bevorzugt 2 bis 50 µg/Tag, noch bevorzugter 5 bis 20 µg/Tag und am meisten bevorzugt 7 bis 10 µg/Tag betragen.

## Revendications

1. Composition pharmaceutique pour utilisation dans le traitement ou la prévention du mélanome chez un sujet humain, dans laquelle la composition comprend une quantité thérapeutiquement efficace de vitamine K2.

2. Composition pharmaceutique pour utilisation selon la revendication 1, dans laquelle ladite composition comprend en outre de la phylloquinone (vitamine K1).

3. Composition pharmaceutique pour utilisation selon la revendication 1 ou 2, dans laquelle ladite vitamine K2 comprend l'une des ménaquinones à chaîne longue MK-7, MK-8, MK-9, MK-10, ou une combinaison de celles-ci.

4. Composition pharmaceutique pour utilisation selon l'une quelconque des revendications 1 à 3, dans laquelle ladite vitamine K2 est administrée en plus de l'apport alimentaire normal en vitamine K sous forme d'aliment fonctionnel, de complément alimentaire ou de préparation pharmaceutique.

5. Composition pharmaceutique pour utilisation selon l'une quelconque des revendications 1 à 4, dans laquelle ladite vitamine K2 est administrée par voie orale dans une plage posologique comprise entre 5 et 5 000 µg/jour, de préférence entre 25 et 1 000 µg/jour, plus préférentiellement entre 50 et 500 µg/jour et le plus préférentiellement entre 100 et 250 µg/jour.

6. Composition pharmaceutique pour utilisation selon l'une quelconque des revendications 1 à 5, dans laquelle la vitamine K2 est administrée par voie topique, en particulier dans une crème, une pommade, un bâton ou une lotion, de préférence dans une plage de concentration comprise entre 0,5 et 100 µg/g de crème, de pommade ou de lotion, plus préférentiellement entre 1 et 20 µg/g et le plus préférentiellement entre 2 et 10 µg/g de crème, de pommade ou de lotion.

7. Composition pharmaceutique pour utilisation selon l'une quelconque des revendications 1 à 6, dans laquelle ledit sujet présente un mauvais bilan en vitamine K, de préférence le sujet présente un mauvais bilan hépatique en vitamine K et/ou un mauvais bilan extrahépatique en vitamine K.

8. Composition pharmaceutique pour utilisation selon l'une quelconque des revendications 1 à 7, dans laquelle la vitamine K2 est destinée à être administrée :
a. en combinaison avec au moins un autre aliment ou complément alimentaire connu pour améliorer la santé de la peau, en particulier des antioxydants ou des acides gras oméga-3, ou
b. en combinaison avec au moins un produit pharmaceutiquement, cosméceutiquement ou cosmétiquement actif, en particulier l'acide hyaluronique, le diméthylaminoéthanol, ou un antibiotique, un corticostéroïde ou un médicament antifongique.

9. Composition pharmaceutique pour utilisation selon l'une quelconque des revendications 1 à 8, dans laquelle ladite composition comprend en outre de la vitamine D, de préférence les doses de vitamine D sont de 2 à 50 µg/jour, plus préférentiellement de 5 à 20 µg/jour, et le plus préférentiellement de 7 à 10 µg/jour.
